# EUROPEAN PATENT APPLICATION

(11) **EP 2 578 217 A1**
(43) Date of publication of application: **10.04.2013**
(21) Application number: 11789419.6
(22) Date of filing: 26.05.2011
(51) Int. Cl.: A61K 31/216, A23L 1/221, A23L 1/226, A23L 1/30, A61K 8/37, A61P 25/02, A61P 25/20, A61Q 5/02, A61Q 5/12, A61Q 19/00, A61Q 19/10

(54) **SYMPATHOLYTIC AGENT, AND COSMETIC, FOOD AND SUNDRY CONTAINING SAME**

(30) Priority: 31.05.2010 JP 2010125331
(71) Applicant: Shiseido Company, Ltd., Chuo-ku Tokyo 104-8010 (JP)
(72) Inventor: MORI, Keiko, Yokohama-shi, Kanagawa 224-8558 (JP); HAZE, Shinichiro, Yokohama-shi, Kanagawa 224-8558 (JP); GOZU, Yoko, Yokohama-shi, Kanagawa 224-8558 (JP); JOICHI, Atsushi, Yokohama-shi, Kanagawa 224-8558 (JP)
(74) Representative: Hart-Davis, Jason
(86) International application number: PCT/JP2011/002953
(87) International publication number: WO 2011/152005

(57) **Abstract**

Disclosed are a sympathetic inhibitor allowing more effective inhibition of activity of a sympathetic nerve, as well as a cosmetic composition, a food, and a sundry article containing the same. Ethyl 4-methoxybenzoate is efficacious for quieting down the activity of the sympathetic nerve. The sympathetic inhibitor, the cosmetic composition, the food and the sundry article containing the same enable more effective inhibition of the activity of the sympathetic nerve.

## Description

### Field of the Invention

The present invention relates to a sympathetic inhibitor for inhibition of activity of a sympathetic nerve, as well as a cosmetic composition, a food, and a sundry article containing the same.

### Related Art

In the modern society, a number of people who upset the balance of an autonomic nervous system caused by stress, culminating in suffering from symptoms of autonomic dystonia are increasing. The autonomic nervous system is composed of two nerve systems of a sympathetic nerve and a parasympathetic nerve, and they keep the balance by controlling each other. When people are nervous or are excited, the sympathetic nerve is predominantly activated, whereas when people are relaxed, the parasympathetic nerve is predominantly activated. However, in the event that this sympathicotonia caused by stress, etc., lasts, the balance with the parasympathetic nerve is upset. This causes various symptoms, such as the persistence of insomnia, blood flow disorder, hot flash, and irritation.
Accordingly, development of the sympathetic inhibitor has been promoted for inhibition of the activity of the sympathetic nerve, thus relieving the aforesaid symptoms (see Patent Document 1).

### Prior Art Document

Patent Document 1: JP 2007-1973344 A

### Summary of the invention

### Problem to be Solved

However, since modern people are always under stress, there is a demand for the development of the sympathetic inhibitor having more superior efficacy.
The present invention has been made in view of the above-explained circumstances, and it is an object of the present invention to provide a sympathetic inhibitor allowing more effective inhibition of activity of the sympathetic nerve, and a cosmetic composition, a food, and a sundry article comprising the same.

### Solution to the Problem

According to an aspect of present invention, there is provided a sympathetic inhibitor comprising ethyl 4-methoxybenzoate.
Further, according to another aspect of the present invention, there is provided a sympathetic inhibitor comprising ethyl 4-methoxybenzoate 0.001 mass% or more and 50 mass% or less.
Moreover, according to yet another aspect of the present invention, there are provided: a cosmetic, a food, and a sundry article each comprising the above sympathetic inhibitor.

### Advantageous Effect of the Invention

Ethyl 4-methoxybenzoate is efficacious for quieting down the activity of the sympathetic nerve. Accordingly, a sympathetic inhibitor according to the present invention, and a cosmetic composition, a food, and a sundry article comprising the same allow effective inhibition of the activity of the sympathetic nerve.

### Brief Description of the Drawings

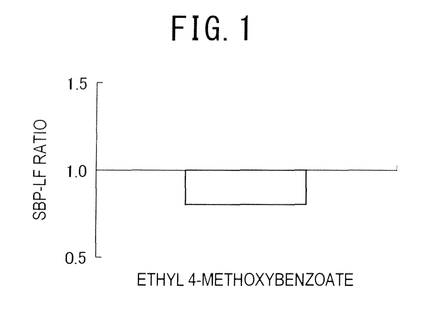

FIG. 1 is a view showing test results.

### Description of Embodiment

A Sympathetic inhibitor according to the present embodiment contains ethyl 4-methoxybenzoate as an active constituent. An aroma given off from the ethyl 4-methoxybenzoate contained in the Sympathetic inhibitor quiets down the activity of a sympathetic nerve when inhaling the aroma. Thus, the sympathetic inhibitor according to the present embodiment allows more effective inhibition of the activity of the sympathetic nerve.

The term "inhibition of the activity of Sympathetic nerve" in the present embodiment means that the activity of the sympathetic nerve quiets down when smelling an aroma given off from the active constituent contained in the sympathetic inhibitor according to the present embodiment, as compared to the activity of the sympathetic nerve when not smelling the aroma given off from the active constituent contained in the sympathetic inhibitor according to the present embodiment.
However, the sympathetic inhibitor according to the present embodiment exerts a sympathetic inhibitory effect, not limited to a case where smelling its aroma. The same sympathetic inhibitory effect is exerted even in a case where the active constituent is absorbed in the body by eating and drinking, and in a case where the active constituent is absorbed through skin.

Hereinafter, a description will be made in detail to the sympathetic inhibitor according to the present embodiment.

### (Ethyl 4-methoxybenzoate)

Ethyl 4-methoxybenzoate is volatile aromatic components contained in flowers, such as orchid and mimosa, etc. Ethyl 4-methoxybenzoate is contained in an extraction essence extracted and obtained from petals of the orchid and the mimosa etc. , are cut into appropriately sized pieces as they are or after having been dried, or from the petals that are pulverized. Alternatively, a fraction produced by reparation and refinement of the extract may be substituted therefor. The extracting method is not limited to any particular one, and typical essential oil extracting methods are applicable, such as distillation method like a steam distillation method or a hot water distillation method, an organic solvent extraction method, and an oil-and-fat adsorptive extraction method, and in particular, the use of the steam distillation method is preferable. In addition, ethyl 4-methoxybenzoate can be synthesized by using the known synthetic method, for example, a commercially available synthetic product may be used.
Ethyl 4-methoxybenzoate is represented by the following chemical formula 1.

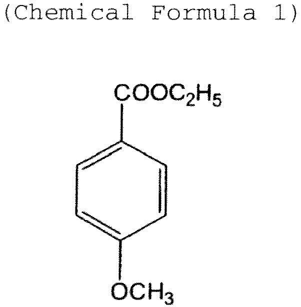

The sympathetic inhibitor according to the present embodiment contains ethyl 4-methoxybenzoate as its active constituent. When inhaling an aroma given off from the active constituent volatilize from the sympathetic inhibitor, the aforesaid sympatho-inhibitory effect is induced. The sympathetic inhibitor may be composed only of the active constituents; but the sympathetic inhibitory may contain any other constituent, such as a diluent, an assistant, and an additive, etc. , up to the amount within a certain range within which exertion of its efficacy is not inhibited. By properly selecting a desired additive, etc., and containing the additive in the sympathetic inhibitor, it is possible to use the sympathetic inhibitor as a cosmetic composition, a medicine, a nonmedical product, a food, a beverage, and a sundry article, etc. Namely, the sympathetic inhibitor may be used as a cosmetic composition, etc., having the sympathetic inhibitory effect.

Meanwhile, adding the sympathetic inhibitor to a specified composition such as a cosmetic composition, etc., enables bestowing the sympathetic inhibitory effect on the composition. Namely, the sympathetic inhibitor can also be added to the cosmetic compositions, etc., to produce the sympathetic inhibitor can the cosmetic compositions, etc., having the Sympathetic inhibitory effect. Moreover, permeating or adhering the sympathetic inhibitor through or to cloth and a sundry article, etc., allows bestowing the sympathetic inhibitory effect on the cloth and the sundry article, etc., as the effective constituent volatilizes from the sympathetic inhibitor permeated through or adhered to the cloth and the sundry article.

While no limitations are put on the containing amount of the active constituent in the sympathetic inhibitor, it is preferable that the containing amount should be equal to or greater than 0.0001 mass% and equal to or smaller than 100 mass%. It is more preferable that the containing amount should be equal to or greater than 0.001 mass% and equal to or smaller than 50 mass%, and it is further preferable that the containing amount should be equal to or greater than 0. 01 mass% and equal to or smaller than 30 mass%.
When the sympathetic inhibitor is added to the composition, such as the cosmetic composition and the medicine, etc., to bestow the sympathetic inhibitory effect thereon, it is preferable that the containing amount of the active constituent in the sympathetic inhibitor to be added should be equal to or greater than 0.0001 mass% and equal to or smaller than 100 mass%. It is more preferable that such a containing amount should be equal to or greater than 0.001 mass% and equal to or smaller than 50 mass%, and it is further preferable that such a containing amount should be equal to or greater than 0.01 mass% and equal to or smaller than 30 mass%.

Further, when sympathetic inhibitor is added to a composition, such as a cosmetic composition or a medicinal drug, to give the sympathetic inhibitory effect thereto, it is preferable that the blending amount of the sympathetic inhibitor in the composition should be equal to or greater than 0.001 mass% and equal to or smaller than 100 mass%. It is especially preferable that such a blending amount should be equal to or greater than 0.003 mass% and equal to or smaller than 30 mass%.
The form of the sympathetic inhibitor according to the present embodiment is not limited to any particular one as long as it can exert the effect according to the present invention, and can be used in an arbitrary form, such as a liquid form, a paste form, a gel form, or a solid form.

Furthermore, the dosage form of the sympathetic inhibitor according to the present embodiment is not limited to any particular one as long as it can exert the effect according to the present invention, and example dosage form thereof are a liquid form, a dust form, a granule form, an aerosol form, a solid form, and a gel form, but the present invention is not limited to those dosage forms.
There is no particular limitation for the cosmetic composition as one of especially suitable forms, but example cosmetic compositions are a fragrance such as perfume, eau du toilette, or au de cologne, cream, milky lotion, skin lotion, massaging gel, massaging cream, foundation, face powder, lip stick, soap, Shampoo and rinse, body shampoo, body rinse, body powder, aerosol, bath additive, etc.

Moreover, when, for example, the sympathetic inhibitor according to the present embodiment is caused to permeate or is added to arbitrary sundry article, such as air freshener, air refresher, aroma candle, incense, stationery product, wallet, bag, and shoes, and arbitrary cloth, such as underwear, dress, hat, stocking, and socks, the above-explained active constituent volatilizes from the permeated or added sympathetic inhibitor, and thus the sympathetic inhibitor effect can be given to such a sundry article, a cloth, etc. The sympathetic inhibitor according to the present embodiment may be caused to permeate or added to the material of the sundry article, or cloth, etc., to produce a product from such a material, or the sympathetic inhibitor according to the present embodiment may be caused to permeate or added to the finished product. In addition, as other available human consumptions, the sympathetic inhibitor can be added to pill, tablet, candy, gum, etc.
Various use forms of the sympathetic inhibitor according to the present embodiment have been exemplified, but the present invention is not limited to those forms, and can be used in an arbitrary form as long as advantageous effects of the present invention can be exerted.

A more detailed explanation will be made below by giving specific examples.
The test samples were prepared by diluting ethyl 4-methoxybenzoate with ethanol to a concentration of 0.1 mass%, and how the aromas of those test samples influenced the sympathetic nerve activity was tested.
A chemical compound commercially available from INOUE PERFUMERY MFG. CO., LTD used for ethyl 4-methoxybenzoate.

An explanation will first be made to a test method adopted for validation of the sympathetic inhibitory effect in the present example.

### (Test Method of Measuring Sympathetic Nerve Activity)

A heart beat interval or blood pressure constantly change in a complex manner even at rest, and is under control of multiple nervous systems representing the sympathetic nerve and the parasympathetic nerve. Thus, it is known that the activity of respective nervous systems can be calculated by analyzing the frequency of a heart beat interval variation (a heart beat interval fluctuation) or a blood pressure variation (a blood pressure fluctuation). A high frequency component of the heat beat interval fluctuation indicates an activity of the sympathetic nerve, whereas a low frequency component thereof indicates a mixed activity of the sympathetic nerve and parasympathetic nerve. In the meanwhile, a low frequency component of the blood pressure fluctuation in systole indicates an activity of the sympathetic nerve (Cardiology, written by Junichiro HAYANO, 34, pp. 347 to 356, 1993). Then, the blood pressure fluctuation in systole is undergone power spectrum analysis using analytical software. The activity of the sympathetic nerve is quantified from the intensity of the low frequency component (SBP-LF), and an influence of an aroma given off from the test sample on the activity of the sympathetic nerve was evaluated.

Six test subjects in their 20's were chosen in the present example. The test content was explained in advance to the test subjects, and the participation in the test was agreed in writing.
The tests were carried out in a room at a constant temperature set to 25 °C and a constant humidity set to 45 %.
A tonometry sensor was fitted at a carpal radius vein of a test subject, and is connected to a patient monitor for continuous measurement of blood pressure. A model BP508 commercially available from OMROM CO., LTD was used for the patient monitor. Continuously measured blood pressure data was taken in a personal computer to undergo power spectrum analysis of the blood pressure fluctuation in systole using automatic nervous system activity analytical software. The activity of the sympathetic nerve was then quantified from the intensity of its low frequency component (SBP-LP). Fluclet WT commercially available from Dainippon Sumitomo Pharma Co., Ltd. was used for the automatic nervous system activity analytical software. The values of SBP-LF between a case where a test subject smells an aroma given from the test sample and a case where the test subject does not smell the aroma given off from the test sample to evaluate what influence of the aroma of the test sample were given to the activity of the Sympathetic nerve.

In the case where the test subject smells the aroma given off from the test sample, the test sample of 20 µl was soaked into cotton, ethanol is fully volatilized, and the cotton was stuck below the nose of the test subject, so that the test subject smelled the aroma with spontaneous respiration. In the case where the test subject did not smell the aroma given off from the test sample, only cotton was stuck under the nose of the test subject.

### (Test Result of Measuring Sympathetic Nerve Activity)

Test results are shown in FIG. 1. The results are shown with a SBP-LF ratio calculated by dividing a SBP-LF value obtained in the case where the test subject smelled the aroma by a SBP-LF value obtained in the case where the test subject did not smell the aroma. When the SBP-LF ratio is larger than 1, it is determined that the activity of the sympathetic nerve was activated, whereas when the SBP-LF ratio is smaller than 1, it is determined that the activity of the sympathetic nerve quieted drown.
As is apparent from FIG. 1, when the test subject smelled the aroma of ethyl 4-methoxybenzoate, the SBP-LF ratio was smaller than 1, and it is determined that the activity of the sympathetic nerve quieted down. Accordingly, it is understood that ethyl 4-methoxybenzoate inhibits the activity of the sympathetic nerve.

Hereinafter, examples of various cosmetic compositions, sundry articles and foods, and further fibers used for a cloth containing the sympathetic inhibitor according to the present embodiment will be explained in detail, but the present invention is not limited to those examples.

| Skin lotion | | mass% |
|---|---|---|
| | (1) Glycerin | 2.0 |
| | (2) Dipropylene glycol | 2.0 |
| | (3) Polyethylene-glycol-60-hydrogenated castor oil | 0.3 |
| | (4) Xylitol | 3 |
| | (5) Ascorbic acid | 0.005 |
| | (6) Edetate trisodium | 0.1 |
| | (7) Dye | 0.1 |
| | (8) Ethyl 4-methoxybenzoate | 0.0001 |
| | (9) Purified water | balance |
| | Total | 100 |

| Milky lotion | mass% |
|---|---|
| (1) Ethyl alcohol | 10.0 |
| (2) Glycerin | 3.0 |
| (3) Butylene glycol | 2.0 |
| (4) Polyethylene glycol | 3.0 |
| (5) Carboxyvinyl polymer | 0.1 |
| (6) Acrylic acid/ alkyl acrylate copolymer | 0.1 |
| (7) Potassium hydroxide | 0.1 |
| (8) Cyclomethicone | 4.0 |
| (9) Squalane | 2.0 |
| (10) Spherical polyethylene | 2.0 |
| (11) Menthol | 0.5 |
| (12) Medicant | 0.1 |
| (13) Paraben | 0.1 |
| (14) Edetate trisodium | 0.1 |
| (15) Pigment | 0.1 |
| (16) Methyl 4-methoxybenzoate | 0.0001 |
| (17) Purified water | balance |
| Total | 100 |

| Cream | mass% |
|---|---|
| (1) Glycerin | 10.0 |
| (2) Butylene glycol | 5.0 |
| (3) Carbomer | 0.1 |
| (4) Potassium hydroxide | 0.2 |
| (5) Stearic acid | 2.0 |
| (6) Glyceryl stearate | 2.0 |
| (7) Glyceryl isostearate | 2.0 |
| (8) Vaseline | 5.0 |
| (9) Preservative agent | 0.1 |
| (10) Antioxidant | 0.1 |
| (11) Ethyle 4-methoxybenzoate | 0.3 |
| (12) Cheating agent | 1.0 |
| (13) Pigments | 0.01 |
| (14) Stearyl alcohol | 2.0 |
| (15) Behenyl alcohol | 2.0 |
| (16) Palm hardened oil | 2.0 |
| (17) Squalane | 10.0 |
| (18) 4-methoxysalicylic acid potassium salts | 3.0 |
| (19) Purified | balance |
| Total | 100 |

| Cream | mass% |
|---|---|
| (1) Glycerin | 3.0 |
| (2) Dipropylene glycol | 7.0 |
| (3) Polyethylene glycol | 3.0 |
| (4) Glyceryl stearate | 3.0 |
| (5) Glyceryl isostearate | 2.0 |
| (6) Stearyl alcohol | 2.0 |
| (7) Behenyl alcohol | 2.0 |
| (8) Liquid paraffin | 7.0 |
| (9) Cyclomethicone | 3.0 |
| (10) Dimethicone | 1.0 |
| (11) Octyl methoxycinnamate | 0.1 |
| (12) Sodium hyaluronate | 0.05 |
| (13) Preservative agent | 0.1 |
| (14) Antioxidant | 0.1 |
| (15) Ethyl 4-methoxybenzoate | 0.4 |
| (16) Chelating agent | 1.0 |
| (17) Pigment | 0.01 |
| (18) Purified | balance |
| Total | 100 |

| Gel | mass% |
|---|---|
| (1) Ethyl alcohol | 10.0 |
| (2) Glycerin | 5.0 |
| (3) Butylene glycol | 5.0 |
| (4) Carbomer | 0.5 |
| (5) knino-methyl-propanol | 0.3 |
| (6) Polyethylene-glycol-60-hydrogenated castor oil | 0.3 |
| (7) Menthol | 0.02 |
| (8) Preservative agent | 0.05 |
| (9) Chelating agent | 1.0 |
| (10) Ethyl 4-methoxybenzoate | 0.1 |
| (11) Purified water | balance |
| Total | 100 |

| Aerosol | mass% |
|---|---|
| (1) Glycerin | 2.0 |
| (2) Dipropylene glycol | 2.0 |
| (3) Polyethylene-glycol-60-hydrogenated | castor oil 0.3 |
| (4) Hydroxypropyl-p-cyclodextrin | 1.0 |
| (5) Preservative agent | 0.1 |
| (6) Chelating agent | 1.0 |
| (7) Pigment | 0.1 |
| (8) Ethyl 4-methoxybenzoate | 0.2 |
| (9) Purified water | 40.0 |
| (10) Liquefied petroleum gas | balance |
| Total | 100 |

| Aerosol | mass% |
|---|---|
| (1) Alcohol | 15.0 |
| (2) Butylene glycol | 2.0 |
| (3) Glycerin | 1.0 |
| (4) Polypropylene glycol-13 decyltetradeceth24 | 0.1 |
| (5) Silver-supporting zeolite | 1.0 |
| (6) Chelating agent | 1.0 |
| (7) Pigment | 0.3 |
| (8) Ethyl 4-methoxybenzoate | 0.15 |
| (9) Liquid petroleum gas | 40.0 |
| (10) Purified water | balance |
| Total | 100 |

| Aerosol | mass% |
|---|---|
| (1) Ethanol | 60.0 |
| (2) Menthyl lactate | 0.1 |
| (3) Sodium lactate | 0.1 |
| (4) Tocopherol acetate | 0.01 |
| (5) Lactic acid | 0.01 |
| (6) Caffeine | 0.01 |
| (7) Fennel essence | 1.0 |
| (8) Witch hazed essence | 1.0 |
| (9) Houttuyuia cordata essence | 1.0 |
| (10) Dipropylene glycol | 1.0 |
| (11) Nitrogen gas | 0.9 |
| (12) Polyoxyethylene-polyoxypropylene-decyltetradecylether | 1.0 |
| (13) Butylene glycol | 2.0 |
| (14) Tocopherol | 0.05 |
| (15) Ethyl 4-methoxybenzoate | 0.1 |
| (16) Polyethylene-glycol-60-hydrogenated | castor oil 0.1 |
| (17) Purified water | balance |
| Total | 100 |

| Shampoo | mass% |
|---|---|
| (1) Lauryl-polyoxyetylene (3) sulfate esther sodium salt (30 % aqueous solution) | 30.0 |
| (2) Lauryl-sulfate esther sodium salt (30 % aqueous solution) | 10.0 |
| (3) Coconut oil fatty acid diethanolamide | 4.0 |
| (4) Glycerin | 1.0 |
| (5) Preservative agent | 0.1 |
| (6) Ethyl 4-methoxybenzoate | 0.5 |
| (7) Pigment | 0.1 |
| (8) Sequestering agent | 0.1 |
| (9) pH adjuster | 0.5 |
| (10) Purified water | valance |
| Total | 100 |

| Rinse | mass% |
|---|---|
| (1) Silicone oil | 3.0 |
| (2) Liquid paraffin | 1.0 |
| (3) Cetyl alcohol | 1.5 |
| (4) Stearyl alcohol | 1.0 |
| (5) Stearyl trimethyl ammonium chloride | 0.7 |
| (6) Glycerin | 3.0 |
| (7) Ethyl 4-methoxybenzoate | 0.5 |
| (8) Pigment | 0.1 |
| (9) Preservative agent | 0.05 |
| (10) Purified water | balance |
| Total | 100 |

| Body shampoo | mass% |
|---|---|
| (1) Lauric acid | 2.5 |
| (2) Myristic acid | 5.0 |
| (3) Palmitic acid | 2.5 |
| (4) Oleic acid | 2.5 |
| (5) Cocoil diethanolamide | 1.0 |
| (6) Glycerin | 20.0 |
| (7) Sodium hydroxide | 3.6 |
| (8) Pigment | 0.1 |
| (9) Ethyl 4-methoxybenzoate | 0.5 |
| (10) Sequestering agent | 0.1 |
| (11) Purified water | balance |
| Total | 100 |

| Fragrance | mass% |
|---|---|
| (1) Alcohol | 75.0 |
| (2) Dipropylene glycol | 5.0 |
| (3) Ethyl 4-methoxybenzoate | 10.0 |
| (4) Antioxidant | 8.0 |
| (5) Pigment | 0.01 |
| (6) Ultraviolet absorber | 0.01 |
| (7) Purified water | balance |
| Total | 100 |

| Room fragrance | mass% |
|---|---|
| (1) Alcohol | 80.0 |
| (2) Antioxidant | 5.0 |
| (3) Ethyl 4-methoxybenzoate | 3.0 |
| (4) 3-metyl-3-methoxybutanol | 5.0 |
| (5) Dibenzylidene sorbitol | 5.0 |
| (6) Purified water | balance |
| Total | 100 |

| Incense | mass% |
|---|---|
| (1) Thunbergii powder | 75.5 |
| (2) Sodium benzoate | 15.5 |
| (3) Ethyl 4-methoxybenzoate | 5.0 |
| (4) Eucalyptus oil | 1.0 |
| (5) Fennel oil | 1.0 |
| (6) Purified water | balance |
| Total | 100 |

| Bath additive | mass% |
|---|---|
| (1) Sodium sulfate | 45.0 |
| (2) Sodium hydrogen carbonate | 45.0 |
| (3) Ethyl 4-methoxybenzoate | 9.0 |
| (4) 2-methoxy benzoic acid methyl | 1.0 |
| Total | 100 |

| Massage gel | mass% |
|---|---|
| (1) Erythritol | 2.0 |
| (2) Caffeine | 5.0 |
| (3) Phellodendron bark extract | 3.0 |
| (4) Glycerin | 50.0 |
| (5) Carboxyvinyl polymer | 0.4 |
| (6) Polyethylene glycol 400 | 30.0 |
| (7) Edetate trisodium | 0.1 |
| (8) Polyoxylene (10) metyl polysiloxane | copolymer 2.0 |
| (9) Squalane | 1.0 |
| (10) Potassium hydroxide | 0.15 |
| (11) Ethyl 4-methoxybenzoate | 1.0 |
| (12) Purified water | balance |
| Total | 100 |

| Massage Cream | mass% |
|---|---|
| (1) Solid paraffin | 5.0 |
| (2) Bees wax | 10.0 |
| (3) Vaseline | 15.0 |
| (4) Liquid paraffin | 41.0 |
| (5) 1,3-butylene glycol | 4.0 |
| (6) Glyceryl monostearate | 2.0 |
| (7) Polyoxylene (20) sorbitan monolaurate ester | 2.0 |
| (8) Borax | 0.2 |
| (9) Caffeine | 2.0 |
| (10) Preservative agent | 0.1 |
| (11) Antioxidant | 0.1 |
| (12) Ethyl 4-methoxybenzoate | 1.0 |
| (13) Purified water | balance |
| Total | 100 |

### Aromatic Fiber

A microcapsule (particle size: equal to or smaller than 50 µm, and the ratio of the sympathetic inhibitor to the micro capsule was 50 mass%) containing the sympathetic inhibitor of the present invention (ethyl 4-methoxybenzoate) was added in and mixed with a cuproammonium cellulose solution (cellulose concentration: 10 mass%, ammonium concentration: 7 mass%, and copper concentration: 3.6 mass%) within a range of 0.1 to 20 mass% relative to the cellulose mass, then fiber spinning was performed in accordance with a normal wet fiber spinning technique, and aromatic fibers were obtained through a refining process and a drying process.

| Granule | mass% |
|---|---|
| (1) Sucralose | 0.1 |
| (2) Ethyl 4-methoxybenzoate | 0.2 |
| (3) Flavoring agent | 5.0 |
| (4) Diluting agent (ceolus) | 10.0 |
| (5) Maltitol | balance |
| Total | 100 |

| Tablet (chewable type) | mass% |
|---|---|
| (1) Inositol | 11.0 |
| (2) Maltitol | 21.0 |
| (3) Sucrose | 0.5 |
| (4) Salmon milt extract (DNA Na) | 0.1 |
| (5) Yeast extract | 0.1 |
| (6) Ethyl 4-methoxybenzoate | 0.1 |
| (7) Flavor | 5.0 |
| (8) Diluting agent | balance |
| Total | 100 |

| Tablet | mass% |
|---|---|
| (1) Lubricant (sucrose fatty acid | ester, etc.) 1.0 |
| (2) Gum arabic solution (5 %) | 2.0 |
| (3) Acidulant | 1.0 |
| (4) Pigment | 0.01 |
| (5) Ethyl 4-methoxybenzoate | 0.1 |
| (6) Carbohydrate (powder sugar or | sorbitol, etc.) balance |
| Total | 100 |

| Candy | mass% |
|---|---|
| (1) Sugar | 50.0 |
| (2) Starch syrup | 47.95 |
| (3) Organic acid | 2.0 |
| (4) Ethyl 4-methoxybenzoate | 0.05 |
| Total | 100 |

| Gum | mass% |
|---|---|
| (1) Sugar | 43.0 |
| (2) Gum base | 30.95 |
| (3) Glucose | 10.0 |
| (4) Starch syrup | 16.0 |
| (5) Ethyl 4-methoxybenzoate | 0.05 |
| Total | 100 |

## Claims

1. A sympathetic inhibitor comprising ethyl 4-methoxybenzoate.

2. A sympathetic inhibitor comprising ethyl 4-methoxybenzoate 0.001 mass% or more and 50 mass% or less.

3. A cosmetic composition comprising the sympathetic inhibitor according to claim 1 or claim 2.

4. A food comprising the sympathetic inhibitor according to claim 1 or claim 2.

5. A sundry article comprising the sympathetic inhibitor according to claim 1 or claim 2.
